# FASCICULE DE BREVET EUROPEEN

(11) **EP 0 855 903 B1**
(45) Date de publication et mention de la délivrance du brevet: **14.08.2002**
(21) Numéro de dépôt: 96933504.1
(22) Date de dépôt: 08.10.1996
(51) Int. Cl.: A61K 7/48, A61K 7/06

(54) **COMPOSITION COSMETIQUE POUR LE TRAITEMENT DES MATIERES KERATINIQUES COMPRENANT AU MOINS UN POLYMERE SILICONE GREFFE ET AU MOINS UNE DISPERTION AQUEUSE DE PARTICULES INSOLUBLES DE POLYMERE NON IONIQUE OU CATIONIQUE**
KOSMETISCHE ZUSAMMENSETZUNG ZUR BEHANDLUNG VON KERATINSUBSTANZEN,DIE MINDESTENS EIN GEPFROPFTES SILICONPOLYMER UND MINDESTENS EINE WÄSSRIGE DISPERSION VON UNLÖSLICHEN PARTIKELN EINES NICHTIONISCHEN ODER KATIONISCHEN POLYMERS ENTHÄLT
COSMETIC COMPOSITION FOR THE TREATMENT OF KERATINOUS MATERIALS COMPRISING AT LEAST A GRAFTED SILICONE POLYMER AND AT LEAST AN AQUEOUS DISPERSION OF INSOLUBLE PARTICLES OF NON IONIC OR CATIONIC POLYMER

(30) Priorité: 18.10.1995 FR 9512235
(43) Date de publication de la demande: 05.08.1998
(73) Titulaire: L'OREAL, 75008 Paris (FR)
(72) Inventeur: DUBIEF, Claude, F-78150 Le Chesnay (FR); DUPUIS, Christine, F-75018 Paris (FR)
(74) Mandataire: Miszputen, Laurent
(86) Numéro de dépôt international: FR9601570
(87) Numéro de publication internationale: WO9714400

(56) Documents cités:
- WO-A-95/03776

## Description

La présente invention a trait à une composition cosmétique ou dermatologique pour le traitement des cheveux comprenant au moins un polymère siliconé greffé, à squelette polysiloxanique greffé par des monomères organiques non-siliconés et au moins une dispersion aqueuse de particules insolubles de polymère non ionique ou cationique.

Il a été proposé d'utiliser des dispersions aqueuses de particules insolubles de polymères dans les compositions pour le maintien de la coiffure.
Cependant, jusqu'à présent, les résultats obtenus ne sont pas encore satisfaisants. En effet, le pouvoir fixant n'est pas encore suffisant, le temps de séchage est long et les propriétés cosmétiques ne sont pas encore satisfaisantes. De plus l'élimination du polymère lors du lavage des cheveux avec un shampooing est difficile.

On connaît également dans l'état de la technique des polymères à squelette polysiloxanique greffé par des monomères organiques non-siliconés. Ils sont choisis préférentiellement parmi ceux décrits dans les demandes EP-A-0582152 et WO 93/23009. Ils sont utilisés en particulier dans les compositions capillaires pour leur propriétés coiffantes.
Les compositions pour le lavage et/ou le soin et/ou le traitement des cheveux contenant dans leur formulation des polymères de coiffage de ce type présentent généralement l'inconvénient de présenter un pouvoir fixant encore insuffisant.

Par pouvoir fixant de la composition on désignera l'aptitude de cette dernière à donner aux cheveux une cohésion telle que la mise en forme initiale de la coiffure est conservée.

On recherche donc encore des compositions ne présentant pas les inconvénients décrits ci-dessus.

La demanderesse a découvert de façon surprenante qu'en associant au moins un polymère siliconé à squelette polysiloxanique greffé par des monomères organiques non-siliconés avec au moins une dispersion de particules insolubles de polymère non ionique ou cationique, on remédiait aux inconvénients cités ci-dessus.

Ces compositions présentent un bon pouvoir fixant et de bonnes propriétés cosmétiques telles que le démêlage et le coiffage ou le brossage des cheveux après application, la douceur, le toucher et le lissage des cheveux.

La composition selon l'invention est donc essentiellement caractérisée par le fait qu'elle comprend dans un milieu cosmétiquement ou dermatologiquement acceptable au moins un polymère siliconé greffé, à squelette polysiloxanique greffé par des monomères organiques non-siliconés et au moins une dispersion de particules insolubles de polymère non ionique ou cationique

Dans ce qui suit, on entend désigner par polymère siliconé, en conformité avec l'acception générale, tous les polymères ou oligomères organosiliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyles notamment en C₁-C₁₀ et en particulier méthyle, les radicaux fluoroalkyles, les radicaux aryles et en particulier phényle, et les radicaux alcényles et en particulier vinyle; d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxyéthylène et/ou polyoxypropylène, les radicaux hydroxyles ou hydroxyalkyles, les groupements aminés substitués ou non, les groupements amides, les radicaux acyloxy ou acyloxyalkyles, les radicaux hydroxyalkylamino ou aminoalkyles, des groupements ammonium quaternaires, des groupements amphotères ou bétaïniques, des groupements anioniques tels que carboxylates, thioglycolates, sulfosuccinates, thiosulfates, phosphates et sulfates, cette liste n'étant bien entendu nullement limitative (silicones dites "organomodifiées").

Selon la présente invention, le ou les polymères greffés siliconés qui doivent être utilisés sont ceux qui comprennent une chaîne principale de silicone (ou polysiloxane (≡Si-O-)ₙ) sur laquelle se trouve greffé, à l'intérieur de ladite chaîne ainsi qu'éventuellement à l'une au moins de ses extrémités, au moins un groupement organique ne comportant pas de silicone.

Ces polymères siliconés peuvent être des produits commerciaux existants, ou encore être obtenus selon tout moyen connu de l'homme de l'art, en particulier par réaction entre (i) une silicone de départ correctement fonctionnalisée sur un ou plusieurs de ces atomes de silicium et (ii) un composé organique non-siliconé lui-même correctement fonctionnalisé par une fonction qui est capable de venir réagir avec le ou les groupements fonctionnels portés par ladite silicone en formant une liaison covalente ; un exemple classique d'une telle réaction est la réaction d'hydrosylilation entre des groupements ≡Si-H et des groupements vinyliques CH₂=CH-, ou encore la réaction entre des groupements thio-fonctionnels -SH avec ces mêmes groupements vinyliques.

Des exemples de polymères siliconés convenant à la mise en oeuvre de la présente invention, ainsi que leur mode particulier de préparation, sont notamment décrits dans les demandes de brevets EP-A- 0 582 152, WO 93/23009 et WO 95/03776.

Selon un mode particulièrement préféré de réalisation de la présente invention, le polymère siliconé mis en oeuvre comprend le résultat de la copolymérisation radicalaire entre d'une part au moins un monomère organique anionique non-siliconé présentant une insaturation éthylénique et/ou un monomère organique hydrophobe non-siliconé présentant une insaturation éthylénique et d'autre part une silicone présentant dans sa chaîne au moins un groupement fonctionnel capable de venir réagir sur lesdites insaturations éthyléniques desdits monomères non-siliconés en formant une liaison covalente, en particulier des groupements thio-fonctionnels.

Selon la présente invention, lesdits monomères anioniques à insaturation éthylénique sont de préférence choisis, seuls ou en mélange, parmi les acides carboxyliques insaturés, linéaires ou ramifiés, éventuellement partiellement ou totalement neutralisés sous la forme d'un sel, ce ou ces acides carboxyliques insaturés pouvant être plus particulièrement l'acide acrylique, l'acide méthacrylique, l'acide maléique, l'anhydride maléique, l'acide itaconique, l'acide fumarique et l'acide crotonique. Les sels convenables sont notamment les sels d'alcalins, d'alcalino-terreux et d'ammonium. On notera que, de même, dans le polymère siliconé greffé final, le groupement organique à caractère anionique qui comprend le résultat de l'(homo) polymérisation radicalaire d'au moins un monomère anionique de type acide carboxylique insaturé peut être, après réaction, post-neutralisé avec une base (soude, ammoniaque,...) pour l'amener sous la forme d'un sel.

Selon la présente invention, les monomères hydrophobes à insaturation éthylénique sont de préférence choisis, seuls ou en mélange, parmi les esters d'acide acrylique alcanols et/ou les esters d'acide méthacrylique d'alcanols. Les alcanols sont de préférence en C₁-C₁₈ et plus particulièrement en C₁-C₁₂. Les monomères préférentiels sont choisis dans le groupe constitué par le (méth)acrylate d'isooctyle, le (méth) acrylate d'isononyle, le 2-éthylhexyl(méth)acrylate, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth) crylate de tridécyle, le (méth)acrylate de stéaryle ou leurs mélanges.

Une famille de polymères siliconés greffés particulièrement bien à la mise en oeuvre de la présente invention est constituée par les polymères siliconés comportant dans leur structure le motif de formule (I) suivant : 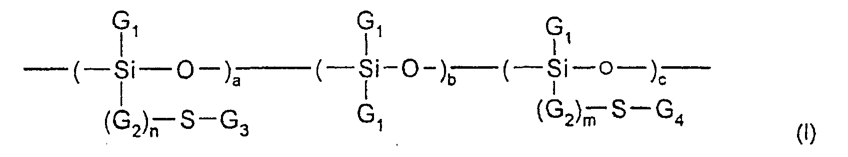 dans lequel les radicaux G₁, identiques ou différents, représentent l'hydrogène ou un radical alkyle en C₁-C₁₀ ou encore un radical phényle ; les radicaux G₂, identiques ou différents, représentent représente un groupe alkylène en C₁-C₁₀; G₃ représente un reste polymérique résultant de l'(homo)polymérisation d'au moins un monomère anionique à insaturation éthylénique ; G₄ représente un reste polymérique résultant de l'(homo)polymérisation d'au moins un monomère d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

De préférence, le motif de formule (I) ci-dessus présente au moins l'une, et encore plus préférentiellement l'ensemble, des caractéristiques suivantes :
- les radicaux G₁ désignent un radical alkyle, de préférence le radical méthyle ;
- n est non nul, et les radicaux G₂ représentent un radical divalent en C₁-C₃, de préférence un radical propylène ;
- G₃ représente un radical polymérique résultant de l'(homo)polymérisation d'au moins un monomère du type acide carboxylique à insaturation éthylénique, de préférence l'acide acrylique et/ou l'acide méthacrylique ;
- G₄ représente un radical polymérique résultant de l'(homo)polymérisation d'au moins un monomère du type (méth)acrylate d'alkyle en C₁-C₁₀, de préférence du type (méth)acrylate d'isobutyle ou de méthyle.

Des exemples de polymères siliconés répondant à la formule (I) sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate de méthyle.

D'autres exemples de polymères siliconés répondant à la formule (I) sont notamment des polydiméthylsiloxanes (PDMS) sur lesquels sont greffés, par l'intermédiaire d'un chaînon de raccordement de type thiopropylène, des motifs polymères du type poly(méth)acrylate d'isobutyle.

De préférence, la masse moléculaire en nombre des polymères siliconés de l'invention varie de 10 000 à 1 000 000 environ, et encore plus préférentiellement de 10 000 à 100 000 environ.

Les polymères siliconés greffés conformes à l'invention sont utilisés de préférence en une quantité allant de 0,01 à 20% en poids du poids total de la composition. Plus préférentiellement, cette quantité varie de 0,1 à 15% en poids et encore plus particulièrement de 0,5 à 10 % en poids.

Les dispersions aqueuses de particules insolubles de polymère non ionique ou cationique utilisables selon l'invention sont généralement obtenues par polymérisation ou copolymérisation en suspension ou en émulsion de monomères selon les procédés bien connus de l'état de la technique (de telles dispersions sont aussi connues sous le nom de "latex"). On peut également obtenir des dispersions aqueuses de polymères en solubilisant ledit polymère dans un solvant organique miscible à l'eau, puis on ajoute de l'eau et enfin on évapore le solvant organique. Ce type de préparation est par exemple décrit dans la demande française n° 2 697 160.

Le diamètre moyen des particules insolubles de polymère est généralement inférieur à 500 nm et de préférence inférieur à 250 nm. La température de transition vitreuse est généralement comprise entre - 30°C et 90°C et de préférence entre 10 et 35°C.

Le polymère de la dispersion aqueuse comprend au moins un monomère choisi par exemple parmi le styrène, le butadiène, l'éthylène, le tétrafluoroéthylène, le propylène, le vinyl toluène, le vinyl propionate, l'alcool vinylique, l'acrylonitrile, le chloroprène, le chlorure de vinyle, l'acétate de vinyle, les uréthannes, l'isoprène, l'isobutène et les esters ou les amides des acides acrylique ou méthacrylique, maléïque, crotonique ou itaconique, les éthers vinyliques, la vinylpyrrolidone, le vinylimidazole, les (méth) acrylate de triméthylammonioéthyle et leurs mélanges.

Les dispersion aqueuses utilisables selon l'invention peuvent provenir de la condensation de monomères ioniques ou non ioniques donnant des polymères non ioniques ou cationiques tels que par exemple les polyesters, les polyamides, les polyuréthannes ou les polyéthers.

Les polymères non ioniques dans les dispersions aqueuses utilisables selon la présente invention sont par exemple choisis parmi les composés suivants :
- les homopolymères d'acétate de vinyle tels que le produit proposé sous le nom de APPRETAN EM par la société HOECHST ou le produit proposé sous le nom de RHODOPAS A 012 par la société RHONE POULENC ;
- les copolymères d'acétate de vinyle et d'ester acrylique tels que le produit proposé sous le nom de RHODOPAS AD 310 de RHONE POULENC ;
- les copolymères d'acétate de vinyle et d'éthylène tels que le produit proposé sous le nom de APPRETAN TV par la société HOECHST ;
- les copolymères d'acétate de vinyle et d'ester maléïque par exemple de maléate de dibutyle tels que le produit proposé sous le nom de APPRETAN MB EXTRA par la société HOECHST ;
- les homopolymères de chlorure de vinyle tels que les produits proposés sous les noms de GEON 460X45, GEON 460X46 et GEON 577 par la société GOODRICH ;
- les cires de polyéthylène tels que les produits proposés sous les dénominations AQUACER 513 et AQUACER 533 par la société BYK CERA ;
- les cires de polyéthylène/polytétrafluoroéthylène tels que les produits proposés sous les dénominations DREWAX D-3750 par la société DREW AMEROID et WAX DISPERSION WD-1077 par la société R.T. NEWEY ;
- les copolymères de polyéthylène et d'anhydride maléïque ;
- les homopolymères d'acrylates d'alkyle et les homopolymères de méthacrylates d'alkyle tels que le produit proposé sous la dénomination MICROPEARL RQ 750 par la société MATSUMOTO ou le produit proposé sous la dénomination LUHYDRAN A 848 S par la société BASF ;
- les copolymères d'esters acryliques tels que par exemple les copolymères d'acrylates d'alkyle et de méthacrylates d'alkyles tels que les produits proposés par la société ROHM&HAAS sous les dénominations PRIMAL ACZ 61 k et EUDRAGIT NE 30 D, par la société BASF sous les dénominations ACRONAL 601, LUHYDRAW LR 8833 ou 8845, par la société HOECHST sous les dénominations APPRETAN N 9213 ou N9212 ;
- les copolymères d'acrylonitrile et d'un monomère non ionique choisi par exemple parmi le butadiène et les (méth)acrylates d'alkyle ; on peut citer les produits proposés sous les dénominations NIPOL LX 531 B par la société NIPPON ZEON ou ceux proposés sous la dénomination CJ 0601 B par la société ROHM & HAAS ;
- les homopolymères de styrène tels que le produit RHODOPAS 5051 proposé par la société RHONE POULENC ;
- les copolymères de styrène et de (méth)acrylate d'alkyle tels que les produits MOWILITH LDM 6911, MOWILITH DM 611 et MOWILITH LDM 6070 proposés par la société HOECHST, les produits RHODOPAS SD 215 et RHODOPAS DS 910 proposés par la société RHONE POULENC, le produit URAMUL SC 70 proposé par la société DSM ;
- les copolymères de styrène, de méthacrylate d'alkyle et d'acrylate d'alkyle tels que le produit DAITISOL SPA proposé par la société WACKHERR ;
- les copolymères de styrène et de butadiène tels que les produits RHODOPAS SB 153 et RHODOPAS SB 012 proposés par la société RHONE POULENC ;
- les copolymères de styrène, de butadiène et de vinylpyridine tels que les produits GOODRITE SB VINYLPYRIDINE 2528X10 et GOODRITE SB VINYLPYRIDINE 2508 proposés par la société GOODRICH ;
- les copolymères de styrène et de vinylpyrrolidone tels que les produits ANTARA 450 et CLOUD 285 proposés par la société ISP ;
- les polyuréthannes tels que les produits proposés sous les dénominations ACRYSOL RM 1020 ou ACRYSOL RM 2020 par la société ROHM & HAAS, les produits URAFLEX XP 401 UZ, URAFLEX XP 402 UZ par la société DSM RESINS ;
- les copolymères d'acrylate d'alkyle et d'uréthanne tels que le produit 8538-33 par la société NATIONAL STARCH ;
- les polyamides tels que le produit ESTAPOR LO 11 proposé par la société RHONE POULENC.

Les dispersions de particules insolubles de polymère cationique comprennent par exemple les polymères suivants :
- les copolymères d'acrylamide et de (méth)acrylate de triméthylammonioéthyle ;
- les copolymères de méthacrylate d'alkyle, d'acrylate d'alkyle et de (méth)acrylate de triméthylammonioéthyle tel que le produit EUDRAGIT RL 30 D proposé par la société ROHM PHARMA.

Les dispersions aqueuses de particules insolubles de polymère particulièrement préférées dans le cadre de l'invention sont les dispersions aqueuses de particules insolubles de polymère non ioniques.

La concentration en poids des particules insolubles de polymère dans les compositions selon l'invention est de préférence comprise entre 0,1 et 50% en poids par rapport au poids total de la composition et de préférence entre 1 et 30%.

Le milieu cosmétiquement ou dermatologiquement acceptable est de préférence constitué par de l'eau ou un mélange d'eau et de solvants cosmétiquement acceptables tels que des monoalcools, des polyalcools, des éthers de glycol ou des esters d'acides gras, qui peuvent être utilisés seuls ou en mélange.

On peut citer plus particulièrement les alcools inférieurs tels que l'éthanol, l'isopropanol, les polyalcools tels que le diéthylèneglycol, les éthers de glycol, les alkyléthers de glycol ou de diéthylèneglycol.

Les polymères siliconés greffés selon l'invention peuvent être dissous dans ledit milieu cosmétiquement acceptable ou utilisés sous forme de dispersion aqueuse de particules insolubles.

La composition de l'invention peut également contenir au moins un additif choisi parmi les épaississants, les esters d'acides gras, les esters d'acides gras et de glycérol, les silicones volatiles, les tensioactifs, les parfums, les conservateurs, les filtres solaires, les protéines, les vitamines, les polymères, les huiles végétales, animales, minérales ou synthétiques et tout autre additif classiquement utilisé dans le domaine cosmétique.

Ces additifs sont présents dans la composition selon l'invention dans des proportions pouvant aller de 0 à 20% en poids par rapport au poids total de la composition. La quantité précise de chaque additif est fonction de sa nature et est déterminée facilement par l'homme de l'art.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous forme de gel, de lait, de crème, de lotion plus ou moins épaissie ou de mousse.

Les compositions selon l'invention sont utilisées comme produits rincés ou comme produits non-rincés notamment pour le lavage, le soin, le conditionnement, le maintien de la coiffure ou la mise en forme des matières kératiniques telles que les cheveux.

Elles sont plus particulièrement des produits de coiffage tels que des lotions de mise en plis, des lotions pour le brushing, des compositions de fixation (laques) et de coiffage. Les lotions peuvent être conditionnées sous diverses formes notamment dans des vaporisateurs, des flacons pompes ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux.

Les compositions peuvent être également des shampooings, des compositions à rincer ou non, à appliquer avant ou après un shampooing, une coloration, une décoloration, une permanente ou un défrisage.

Lorsque la composition selon l'invention est conditionnée sous forme d'aérosol en vue d'obtenir une laque ou une mousse aérosol, elle comprend au moins un agent propulseur qui peut être choisi parmi les hydrocarbures volatils tels que le n-butane, le propane, l'isobutane, le pentane, un hydrocarbure chloré et/ou fluoré et leurs mélanges. On peut également utiliser en tant qu'agent propulseur le gaz carbonique, le protoxyde d'azote, le diméthyléther, l'azote, l'air comprimé et leurs mélanges.

L'invention a encore pour objet un procédé de traitement des matières kératiniques telles que les cheveux consistant à appliquer sur ceux-ci une composition telle que définie précédemment puis à effectuer éventuellement un rinçage à l'eau.

L'invention va être maintenant plus complètement illustrée à l'aide des exemples suivants qui ne sauraient être considérés comme la limitant aux modes de réalisation décrits. Dans tout ce qui suit, MA signifie Matière Active.

### EXEMPLE 1 Spray de coiffage en aérosol

- Polymère siliconé greffé de formule (I) de structure polyméthyl/méthylsiloxane à groupements propyl thio-3 acide polyméthacrylique et groupements propyl thio-3 polyméthacrylate de méthyle 3 g
- Homopolymère d'acétate de vinyle en dispersion aqueuse à 50% de MA proposé sous la dénomination APPRETAN EM par la société HOECHST 4g MA
- Aminométhylpropanol qsp neutralisation à 100% du polymère siliconé greffé
- Diméthyléther 30 g
- Eau qsp 100 g

La composition est pressurisée en aérosol.

On a appliqué cette composition sur des cheveux séchés, les cheveux présentent alors un bon toucher, un bon maintien et une bonne tenue dans le temps.
La composition s'élimine facilement au shampooing.

### EXEMPLE 2 Spray de coiffage en aérosol

- Polymère siliconé greffé de formule (i) de structure polyméthyl/méthylsiloxane à groupements propyl thio-3 acide polyméthacrylique et groupements propyl thio-3 polyméthacrylate de méthyle 4 g
- Copolymère de styrène et de butadiène en dispersion aqueuse à 50% de MA proposé sous la dénomination RHODOPAS SB 012 par la société RHONE POULENC 2 g MA
- Aminométhylpropanol qsp neutralisation à 100% du polymère siliconé greffé
- Diméthyléther 30 g
- Eau qsp 100 g

La composition est pressurisée en aérosol.

On a appliqué cette composition sur des cheveux séchés, les cheveux présentent alors un bon toucher, un bon maintien et une bonne tenue dans le temps.
La composition s'élimine facilement au shampooing.

## Revendications

1. Composition cosmétique ou dermatologique destinée au traitement des cheveux, **caractérisée par le fait qu'**elle comprend dans un milieu cosmétiquement ou dermatologiquement acceptable au moins un polymère siliconé greffé, à squelette polysiloxanique greffé par des monomères organiques non-siliconés et au moins une dispersion aqueuse de particules insolubles de polymère non ionique ou cationique.

2. Composition selon la revendication 1, **caractérisée par le fait que** le polymère siliconé greffé comprend une chaîne principale de polysiloxane sur laquelle se trouve greffé, à l'intérieur de ladite chaîne ainsi qu'éventuellement à l'une au moins de ses extrémités, au moins un groupement organique ne comportant pas de silicone.

3. Composition selon la revendication 1 ou 2, **caractérisée par le fait que** le polymère siliconé greffé est susceptible d'être obtenu par copolymérisation radicalaire entre d'une part au moins un monomère organique anionique non-siliconé présentant une insaturation éthylénique et/ou un monomère organique hydrophobe non-siliconé présentant une insaturation éthylénique et d'autre part un polysiloxane présentant dans sa chaîne au moins un, et de préférence plusieurs, groupements fonctionnels capables de venir réagir sur lesdites insaturations éthyléniques desdits monomères non-siliconés.

4. Composition selon la revendication 3, **caractérisée par le fait que** le monomère organique anionique à insaturation éthylénique est choisi, seul ou sous forme de mélange de monomères, parmi les acides carboxyliques insaturés, linéaires ou ramifiés.

5. Composition selon la revendication 4, **caractérisée par le fait que** le monomère organique anionique à insaturation éthylénique est choisi, seul ou sous forme de mélange de monomères, parmi l'acide acrylique, l'acide méthacrylique, l'acide maléique, l'anhydride maléique, l'acide itaconique, l'acide fumarique et l'acide crotonique ou leurs sels d'alcalins, d'alcalino-terreux ou d'ammonium, ou leurs mélanges.

6. Composition selon la revendications 3, **caractérisée par le fait que** le monomère organique hydrophobe à insaturation éthylénique est choisi, seul ou en mélange de monomères, parmi les esters d'acide acrylique d'alcanol et/ou les esters d'acide méthacrylique d'alcanol, de préférence l'alcanol étant en C₁-C₁₈.

7. Composition selon la revendications 6, **caractérisée par le fait que** le monomère organique hydrophobe à insaturation éthylénique est choisi, seul ou en mélange de monomères dans le groupe constitué par le (méth)acrylate d'isooctyle, le (méth)acrylate d'isononyle, le 2-éthylhexyl(méth)acrylate, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth)acrylate de tridécyle, le (méth)acrylate de stéaryle.

8. Composition selon l'une quelconque des revendications 1 à 7, **caractérisée par le fait que** le polymère siliconé greffé comprend sur la chaîne silicone principale, au moins un groupement organique à caractère anionique obtenu par l'(homo)polymérisation radicalaire d'au moins un monomère anionique de type acide carboxylique insaturé partiellement ou totalement neutralisé sous la forme d'un sel.

9. Composition selon l'une quelconque des revendications 1 à 8, **caractérisée par le fait que** le polymère siliconé greffé est choisi parmi les polymères siliconés comportant dans leur structure le motif de formule (I) suivant : 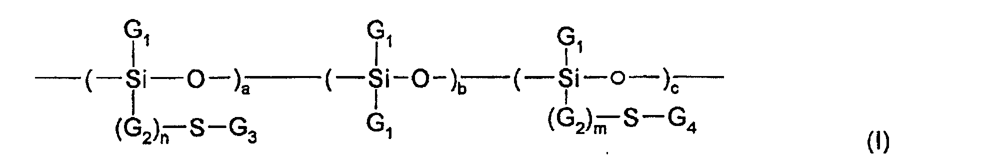 dans lequel les radicaux G₁, identiques ou différents, représentent l'hydrogène ou un radical alkyle en C₁-C₁₀ ou encore un radical phényle ; les radicaux G₂, identiques ou différents, représentent un groupe alkylène en C₁-C₁₀ ; G₃ représente un reste polymérique résultant de l'(homo)polymérisation d'au moins un monomère anionique à insaturation éthylénique ; G₄ représente un reste polymérique résultant de l'(homo)polymérisation d'au moins un monomère d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

10. Composition selon la revendication 9, **caractérisée par le fait que** le motif de formule (I) présente au moins l'une des caractéristiques suivantes :
- les radicaux G₁ désignent un radical alkyle en C₁-C₁₀ ;
- n est non nul, et les radicaux G₂ représentent un radical divalent en C₁-C₃ ;
- G₃ représente un radical polymérique résultant de l'(homo)polymérisation d'au moins un monomère du type acide carboxylique à insaturation éthylénique ;
- G₄ représente un radical polymérique résultant de l'(homo)polymérisation d'au moins un monomère du type (méth)acrylate d'alkyle en C₁-C₁₀.

11. Composition selon la revendication 9 ou 10, **caractérisée par le fait que** le motif de formule (1) présente simultanément les caractéristiques suivantes :
- les radicaux G₁ désignent un radical méthyle ;
- n est non nul, et les radicaux G₂ représentent un radical propylène ;
- G₃ représente un radical polymérique résultant de l'(homo)polymérisation d'au moins l'acide acrylique et/ou l'acide méthacrylique ;
- G₄ représente un radical polymérique résultant de l'(homo)polymérisation d'au moins le (méth)acrylate de méthyle.

12. Composition selon l'une quelconque des revendications 1 à 11, **caractérisée par le fait que** la masse moléculaire en nombre du polymère siliconé greffé varie de 10 000 à 1 000 000 environ, et encore plus préférentiellement de 10 000 à 100 000 environ.

13. Composition selon l'une quelconque des revendications précédentes, **caractérisé par le fait que** le ou les polymères greffés siliconés sont présents dans des concentrations allant de 0,01 à 20% en poids par rapport au poids total de la composition, de préférence de 0,1 à 15% en poids et plus particulièrement de 0,5 à 10 % en poids.

14. Composition selon l'une quelconque des revendications précédentes, **caractérisée par le fait que** le polymère de la dispersion aqueuse comprend au moins un monomère choisi parmi le styrène, le butadiène, l'éthylène, le propylène, le vinyl toluène, le vinyl propionate, l'alcool vinylique, l'acrylonitrile, le chloroprène, l'acétate de vinyle, les uréthannes, l'isoprène, l'isobutène et les esters ou les amides des acides acrylique ou méthacrylique, maléïque, crotonique ou itaconique, l'éther vinylique, la vinylpyrrolidone, le vinylimidazole les (méth)acrylate de triméthylammonioéthyle et leurs mélanges.

15. Composition selon l'une quelconque des revendications précédentes, **caractérisée par le fait que** le polymère non ionique de la dispersion aqueuse est choisi parmi les polyesters, les polyamides, les polyuréthannes et les polyéthers.

16. Composition selon l'une quelconque des revendications précédentes, **caractérisée par le fait que** le polymère non ionique de la dispersion aqueuse est choisi parmi :
- les homopolymères d'acétate de vinyle ;
- les copolymères d'acétate de vinyle et d'ester acrylique ;
- les copolymères d'acétate de vinyle et d'éthylène ;
- les copolymères d'acétate de vinyle et d'ester maléïque ;
- les homopolymères de chlorure de vinyle ;
- les cires de polyéthylène ;
- les cires de polyéthylène/polytétrafluoroéthylène ;
- les copolymères de polyéthylène et d'anhydride maléïque ;
- les homopolymères d'acrylates d'alkyle et les homopolymères de méthacrylates d'alkyle ;
- les copolymères d'esters acryliques tels que par exemple les copolymères d'acrylates d'alkyle et de méthacrylates d'alkyles ;
- les copolymères d'acrylonitrile et d'un monomère non ionique choisi par exemple parmi le butadiène et les (méth)acrylates d'alkyle ;
- les homopolymères de styrène ;
- les copolymères de styrène et de (méth)acrylate d'alkyle ;
- les copolymères de styrène, de méthacrylate d'alkyle et d'acrylate d'alkyle ;
- les copolymères de styrène et de butadiène ;
- les copolymères de styrène, de butadiène et de vinylpyridine ;
- les copolymères de styrène et de vinylpyrrolidone ;
- les copolymères d'acrylate d'alkyle et d'uréthanne.

17. Composition selon l'une quelconque des revendications 1 à 14, **caractérisée par le fait que** le polymère cationique de la dispersion aqueuse est choisi parmi :
- les copolymères d'acrylamide et de (méth)acrylate de triméthylammonioéthyle ;
- les copolymères de méthacrylate d'alkyle, d'acrylate d'alkyle et de (méth)acrylate de triméthylammonioéthyle.

18. Composition selon l'une quelconque des revendications précédentes, **caractérisée par le fait que** la concentration en poids des particules insolubles de polymère est comprise entre 0,1 et 50% par rapport au poids total de la composition.

19. Composition selon l'une quelconque des revendications précédentes, **caractérisée par le fait que** la concentration en poids des particules insolubles de polymère est comprise entre 1 et 30% par rapport au poids total de la composition.

20. Composition selon l'une quelconque des revendications précédentes, **caractérisée par le fait qu'**elle contient en plus au moins un additif choisi dans le groupe constitué par les épaississants, les esters d'acides gras, les esters d'acides gras et de glycérol, les silicones, les tensioactifs, les parfums, les conservateurs, les filtres solaires, les protéines, les vitamines, les polymères, les huiles végétales, animales, minérales ou synthétiques et tout autre additif classiquement utilisé dans le domaine cosmétique.

21. Composition selon l'une quelconque des revendications précédentes, **caractérisée par le fait que** le milieu cosmétiquement ou dermatologiquement acceptable est constitué par de l'eau ou un mélange d'eau et d'au moins un solvant cosmétiquement acceptable.

22. Composition selon la revendication 21, **caractérisée par le fait que** les solvants cosmétiquement acceptables sont choisis dans le groupe constitué par les monoalcools, les polyalcools, les éthers de glycol, les esters d'acides gras et leurs mélanges.

23. Composition selon l'une quelconque des revendications précédentes, **caractérisée par le fait qu'**elle se présente sous forme de gel, de lait, de crème, de lotion plus ou moins épaissie ou de mousse.

24. Composition selon l'une quelconque des revendications 1 à 23, **caractérisée par le fait qu'**elle est un produit de coiffage.

25. Composition selon l'une quelconque des revendications 1 à 24, **caractérisée par le fait qu'**elle est un produit capillaire choisi dans le groupe constitué par des shampooings ; des produits capillaires à rincer ou non, à appliquer avant ou après un shampooing, une coloration, une décoloration, une permanente ou un défrisage.

26. Composition selon l'une quelconque des revendications 1 à 25, **caractérisée par le fait qu'**elle est conditionnée sous forme de vaporisateur, de flacon pompe ou bien dans un récipient aérosol en vue d'obtenir un spray, une laque ou une mousse.

27. Composition selon l'une quelconque des revendications précédentes, **caractérisée par le fait que** le polymère siliconé greffé est dissous dans le milieu cosmétiquement ou dermatologiquement acceptable ou utilisé sous forme de dispersion aqueuse de particules insolubles.

28. Procédé non-thérapeutique de traitement des cheveux, **caractérisé par le fait qu'**il consiste à appliquer sur lesdits cheveux une composition telle que définie selon l'une quelconque des revendications 1 à 27 puis à effectuer éventuellement un rinçage à l'eau.

## Patentansprüche

1. Kosmetische oder dermatologische Zusammensetzung, die zur Behandlung von Haaren vorgesehen ist, **dadurch gekennzeichnet, daß** sie in einem kosmetisch oder dermatologisch akzeptablen Medium mindestens ein gepfropftes Siliconpolymer mit Polysiloxangerüst, auf das nicht siliconhaltige, organische Monomere gepfropft sind, und mindestens eine wäßrige Dispersion von unlöslichen Partikeln eines nichtionischen oder kationisches Polymers enthält.

2. Zusammensetzung nach Anspruch 1, **dadurch gekennzeichnet, daß** das gepfropfte Siliconpolymer eine Siliconhauptkette aufweist, auf die in der Kette sowie gegebenenfalls an mindestens einem ihrer Enden mindestens eine organische Gruppe gepfropft ist, die kein Silicon enthält.

3. Zusammensetzung nach Anspruch 1 oder 2, **dadurch gekennzeichnet, daß** das gepfropfte Siliconpolymer durch radikalische Polymerisation mindestens eines nicht siliconhaltigen, anionischen organischen Monomers mit ethylenischer Doppelbindung und/oder eines nicht siliconhaltigen, hydrophoben organischen Monomers mit ethylenischer Doppelbindung und eines Polysiloxans hergestellt werden kann, das in seiner Kette mindestens eine und vorzugsweise mehrere funktionelle Gruppen aufweist, die mit den ethylenischen Doppelbindungen der nicht siliconhaltigen Monomere reagieren können.

4. Zusammensetzung nach Anspruch 3, **dadurch gekennzeichnet, daß** die anionischen organischen Monomere mit ethylenischer Doppelbindung einzeln oder in Form von Monomerengemischen unter den geradkettigen oder verzweigten, ungesättigten Carbonsäuren ausgewählt sind.

5. Zusammensetzung nach Anspruch 4, **dadurch gekennzeichnet, daß** die anionischen organischen Monomere mit ethylenischer Doppelbindung einzeln oder in Form von Monomerengemischen unter Acrylsäure, Methacrylsäure, Maleinsäure, Maleinsäureanhydrid, Itaconsäure, Fumarsäure, Crotonsäure oder deren Alkali-, Erdalkali- oder Ammoniumsalzen oder deren Gemischen ausgewählt sind.

6. Zusammensetzung nach Anspruch 3, **dadurch gekennzeichnet, daß** das hydrophobe organische Monomer mit ethylenischer Doppelbindung einzeln oder in Form von Monomerengemischen unter den Estern von Acrylsäure und einem Alkanol und/oder den Estern von Methacrylsäure und einem Alkanol ausgewählt sind, wobei das Alkanol vorzugsweise 1 bis 18 Kohlenstoffatome aufweist.

7. Zusammensetzung nach Anspruch 6, **dadurch gekennzeichnet, daß** das hydrophobe, organische Monomer mit ethylenischer Doppelbindung einzeln oder in Form von Monomerengemischen unter Isooctyl(meth)acrylat, Isononyl(meth)acrylat, 2-Ethylhexyl(meth)-acrylat, Lauryl(meth)acrylat, Isopentyl(meth)acrylat, n-Butyl(meth)-acrylat, Isobutyl(meth)acrylat, Methyl(meth)acrylat, t-Butyl(meth)-acrylat, Tridecyl(meth)acrylat und Stearyl(meth)acrylat ausgewählt ist.

8. Zusammensetzung nach einem der Ansprüche 1 bis 7, **dadurch gekennzeichnet, daß** das gepfropfte Siliconpolymer in der Siliconhauptkette mindestens eine organische Gruppe mit anionischen Eigenschaften aufweist, die durch radikalische (Homo)polymerisation mindestens eines anionischen Monomers vom Typ einer ungesättigten, zum Teil oder vollständig in Form eines Salzes neutralisierten Carbonsäure hergestellt wird.

9. Zusammensetzung nach einem der Ansprüche 1 bis 8, **dadurch gekennzeichnet, daß** das gepfropfte Siliconpolymer unter den Siliconpolymeren ausgewählt ist, die in ihrer Struktur die Einheit der folgenden Formel (I) enthalten: 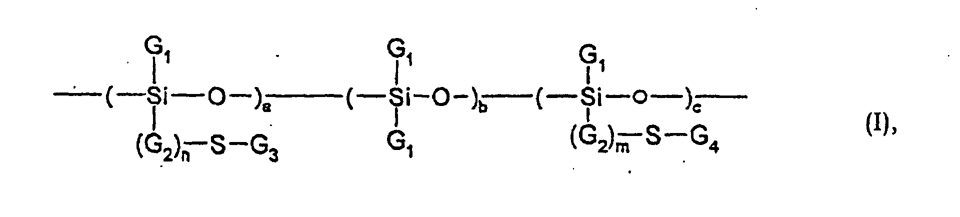 worin die Gruppen G₁, die identisch oder voneinander verschieden sind, Wasserstoff, C₁₋₁₀-Alkyl oder Phenyl; die Gruppen G₂, die identisch oder voneinander verschieden sind, C₁₋₁₀-Alkylen; G₃ eine Polymergruppe, die aus der (Homo)polymerisation mindestens eines anionischen Monomers mit ethylenischer Doppelbindung resultiert; G₄ eine Polymergruppe, die aus der (Homo)polymerisation mindestens eines hydrophoben Monomers mit ethylenischer Doppelbindung resultiert; m und n 0 oder 1; a 0 oder eine ganze Zahl im Bereich von 1 bis 50; b eine ganze Zahl im Bereich von 10 bis 350 und c 0 oder eine ganze Zahl von 1 bis 50 bedeuten, mit der Maßgabe, daß einer der Parameter a oder c von 0 verschieden ist.

10. Zusammensetzung nach Anspruch 9, **dadurch gekennzeichnet, daß** die Einheit der Formel I mindestens eine der folgenden Eigenschaften aufweist:
- die Gruppen G₁ bedeuten eine C₁₋₁₀-Alkylgruppe;
- n ist nicht 0 und die Gruppen G₂ bedeuten eine zweiwertige Gruppe mit 1 bis 3 Kohlenstoffatomen;
- G₃ bedeutet eine Polymergruppe, die bei der (Homo)polymerisation mindestens eines Monomers vom Typ einer Carbonsäure mit ethylenischer Doppelbindung gebildet wird; und
- G₄ bedeutet eine Polymergruppe, die bei der (Homo)polymerisation mindestens eines Monomers vom Typ C₁₋₁₀-Alkyl(meth)acrylat entsteht.

11. Zusammensetzung nach Anspruch 9 oder 10, **dadurch gekennzeichnet, daß** die Einheit der Formel I alle folgenden Eigenschaften aufweist:
- die Gruppen G₁ bedeuten Methyl;
- n ist nicht 0 und die Gruppen G₂ bedeuten Propylen;
- G₃ bedeutet eine Polymergruppe, die bei der (Homo)polymerisation von zumindest Acrylsäure und/oder Methacrylsäure entsteht;
- G₄ bedeutet eine Polymergruppe, die bei der (Homo)polymerisation von zumindest Methyl(meth)acrylat entsteht.

12. Zusammensetzung nach einem der Ansprüche 1 bis 11, **dadurch gekennzeichnet, daß** das Zahlenmittel des Molekulargewichts des gepfropften Siliconpolymers im Bereich von etwa 10 000 bis 1 000 000 und noch bevorzugter von etwa 10 000 bis 100 000 liegt.

13. Zusammensetzung nach einem der vorhergehenden Ansprüche, **dadurch gekennzeichnet, daß** das oder die gepfropften Siliconpolymere in Konzentrationen von 0,01 bis 20 Gew.-%, bezogen auf das Gesamtgewicht der Zusammensetzung, vorzugsweise 0,1 bis 15 Gew.-% und insbesondere im Bereich von 0,5 bis 10 Gew.-% verwendet werden.

14. Zusammensetzung nach einem der vorhergehenden Ansprüche, **dadurch gekennzeichnet, daß** das Polymer der wäßrigen Dispersion mindestens ein Monomer enthält, das unter Styrol, Butadien, Ethylen, Tetrafluorethylen, Propylen, Vinyltoluol, Vinylpropionat, Vinylalkohol, Acrylnitril, Chloropren, Vinylacetat, Urethanen, Isopren, Isobuten, den Estern oder Amiden von Acrylsäure oder Methacrylsäure, Maleinsäure, Crotonsäure oder Itaconsäure, Vinylethern, Vinylpyrrolidon, Vinylimidazol, Trimethylammoniethyl(meth)acrylaten und ihren Gemischen ausgewählt ist.

15. Zusammensetzung nach einem der vorhergehenden Ansprüche, **dadurch gekennzeichnet, daß** das nichtionische Polymer der wäßrigen Dispersion unter den Polyestern, Polyamiden, Polyurethanen und Polyethern ausgewählt ist.

16. Zusammensetzung nach einem der vorhergehenden Ansprüche, **dadurch gekennzeichnet, daß** das nichtionische Polymer der wäßrigen Dispersion ausgewählt ist unter:
- Homopolymeren von Vinylacetat;
- Copolymeren von Vinylacetat und einem Acrylester;
- Copolymeren von Vinylacetat und Ethylen;
- Copolymeren von Vinylacetat und einem Maleinsäureester;
- Homopolymeren von Vinylchlorid;
- Polyethylenwachsen;
- Wachsen von Polyethylen und Polytetrafluorethylen;
- Copolymeren von Polyethylen und Maleinsäureanhydrid;
- Homopolymeren von Alkylacrylaten und Homopolymeren von Alkylmethacrylaten;
- Copolymeren von Acrylestern, wie beispielsweise den Copolymeren von Alkylacrylaten und Alkylmethacrylaten;
- Copolymeren von Acrylnitril und einem nichtionischen Monomer, das beispielsweise unter Butadien und Alkyl(meth)-acrylaten ausgewählt ist;
- Homopolymeren von Styrol;
- Copolymeren von Styrol und Alkyl(meth)acrylat;
- Copolymeren von Styrol, Alkylmethacrylat und Alkylacrylat;
- Copolymeren von Styrol und Butadien;
- Copolymeren von Styrol, Butadien und Vinylpyridin;
- Copolymeren von Styrol und Vinylpyrrolidon;
- Copolymeren von Alkylacrylat und Urethan.

17. Zusammensetzung nach einem der Ansprüche 1 bis 14, **dadurch gekennzeichnet, daß** das kationische Polymer der wäßrigen Dispersion ausgewählt ist unter:
- Copolymeren von Acrylamid und Trimethylammonioethyl(meth)-acrylat;
- Copolymeren von Alkylmethacrylat, Alkylacrylat und Trimethylammonioethyl(meth)acrylat.

18. Zusammensetzung nach einem der vorhergehenden Ansprüche, **dadurch gekennzeichnet, daß** die Konzentration der unlöslichen Polymerpartikel im Bereich von 0,1 bis 50 Gew.-%, bezogen auf das Gesamtgewicht der Zusammensetzung, liegt.

19. Zusammensetzung nach einem der vorhergehenden Ansprüche, **dadurch gekennzeichnet, daß** die Konzentration der unlöslichen Polymerpartikel im Bereich von 1 bis 30 Gew.-%, bezogen auf das Gesamtgewicht der Zusammensetzung, liegt.

20. Zusammensetzung nach einem der vorhergehenden Ansprüche, **dadurch gekennzeichnet, daß** sie ferner mindestens einen Zusatzstoff enthält, der unter den Verdickungsmitteln, Fettsäureestern, Estern von Fettsäuren und Glycerin, Siliconen, grenzflächenaktiven Stoffen, Parfums, Konservierungsmitteln, Sonnenschutzfiltern, Proteinen, Vitaminen, Polymeren, pflanzlichen Ölen, tierischen Ölen, Mineralölen oder synthetischen Ölen oder beliebigen weiteren herkömmlich in der Kosmetik verwendeten Zusatzstoffen ausgewählt ist.

21. Zusammensetzung nach einem der vorhergehenden Ansprüche, **dadurch gekennzeichnet, daß** das kosmetisch oder dermatologisch akzeptable Medium aus Wasser oder einem Gemisch von Wasser und mindestens einem kosmetisch akzeptablen Lösungsmittel besteht.

22. Zusammensetzung nach Anspruch 21, **dadurch gekennzeichnet, daß** die kosmetisch akzeptablen Lösungsmittel unter den Monoalkoholen, Polyalkoholen, Glykolethern, Fettsäureestern und deren Gemischen ausgewählt sind.

23. Zusammensetzung nach einem der vorhergehenden Ansprüche, **dadurch gekennzeichnet, daß** sie als Gel, Milch, Creme, mehr oder weniger dickflüssige Lotion oder Schaum vorliegt.

24. Zusammensetzung nach einem der Ansprüche 1 bis 23, **dadurch gekennzeichnet, daß** es sich um ein Produkt für die Frisur handelt.

25. Zusammensetzung nach einem der Ansprüche 1 bis 24, **dadurch gekennzeichnet, daß** es sich um ein Produkt für das Haar handelt, das unter den Haarwaschmitteln und Produkten für das Haar, die ausgespült werden oder im Haar verbleiben, vor oder nach einer Haarwäsche, Färbung, Entfärbung, Dauerwelle oder Entkräuselung aufgetragen werden, ausgewählt ist.

26. Zusammensetzung nach einem der Ansprüche 1 bis 25, **dadurch gekennzeichnet, daß** sie zur Bildung eines Sprays, eines Lacks oder eines Schaums in einem Zerstäuber, Pumpflakon oder Aerosolbehälter konfektioniert ist.

27. Zusammensetzung nach einem der vorhergehenden Ansprüche, **dadurch gekennzeichnet, daß** das gepfropfte Siliconpolymer in dem kosmetisch oder dermatologisch akzeptablen Medium gelöst ist oder in Form einer wäßrigen Dispersion von Partikeln verwendet wird.

28. Verfahren zur nicht therapeutischen Behandlung des Haares, **dadurch gekennzeichnet, daß** es darin besteht, auf die Haare eine Zusammensetzung nach einem der Ansprüche 1 bis 27 aufzutragen und gegebenenfalls mit Wasser zu spülen.

## Claims

1. Cosmetic or dermatological composition intended for treating the hair, **characterized in that** it comprises, in a cosmetically or dermatologically acceptable medium, at least one grafted silicone polymer with a polysiloxane skeleton grafted with non-silicone organic monomers and at least one aqueous dispersion of insoluble particles of nonionic or cationic polymer.

2. Composition according to Claim 1, **characterized in that** the grafted silicone polymer comprises a main polysiloxane chain on which is grafted, inside the said chain as well as, optionally, on at least one of its ends, at least one organic group containing no silicone.

3. Composition according to Claim 1 or 2, **characterized in that** the grafted silicone polymer can be obtained by radical copolymerization between, on the one hand, at least one non-silicone anionic organic monomer having ethylenic unsaturation and/or a non-silicone hydrophobic organic monomer having ethylenic unsaturation, and, on the other hand, a polysiloxane having in its chain at least one, and preferably several, functional groups capable of reacting with the said ethylenic unsaturations of the said non-silicone monomers.

4. Composition according to Claim 3, **characterized in that** the anionic organic monomer containing ethylenic unsaturation is chosen, alone or in the form of a monomer mixture, from linear or branched unsaturated carboxylic acids.

5. Composition according to Claim 4, **characterized in that** the anionic organic monomer containing ethylenic unsaturation is chosen, alone or in the form of a monomer mixture, from acrylic acid, methacrylic acid, maleic acid, maleic anhydride, itaconic acid, fumaric acid and crotonic acid or alkali metal, alkaline-earth metal or ammonium salts thereof, or mixtures thereof.

6. Composition according to Claim 3, **characterized in that** the hydrophobic organic monomer containing ethylenic unsaturation is chosen, alone or as a monomer mixture, from acrylic acid esters of an alkanol and/or methacrylic acid esters of an alkanol, the alkanol preferably being C₁-C₁₈.

7. Composition according to Claim 6, **characterized in that** the hydrophobic organic monomer containing ethylenic unsaturation is chosen, alone or as a monomer mixture, from the group consisting of isooctyl (meth)acrylate, isononyl (meth)acrylate, 2-ethylhexyl (meth)acrylate, lauryl (meth)acrylate, isopentyl (meth)acrylate, n-butyl (meth)acrylate, isobutyl (meth)acrylate, methyl (meth)acrylate, tert-butyl (meth)acrylate, tridecyl (meth)acrylate and stearyl (meth)acrylate.

8. Composition according to any one of Claims 1 to 7, **characterized in that** the grafted silicone polymer comprises, on the main silicone chain, at least one organic group of anionic nature obtained by radical (homo)polymerization of at least one anionic monomer of unsaturated carboxylic acid type, partially or totally neutralized in the form of a salt.

9. Composition according to any one of Claims 1 to 8, **characterized in that** the grafted silicone polymer is chosen from silicone polymers containing in their structure the unit of formula (I) below: 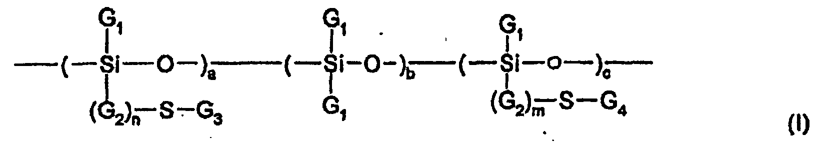 in which the radicals G₁, which may be identical or different, represent hydrogen or a C₁-C₁₀ alkyl radical or alternatively a phenyl radical; the radicals G₂, which may be identical or different, represent a C₁-C₁₀ alkylene group; G₃ represents a polymer residue resulting from the (homo)polymerization of at least one anionic monomer containing ethylenic unsaturation; G₄ represents a polymer residue resulting from the (homo)polymerization of at least one hydrophobic monomer containing ethylenic unsaturation; m and n are equal to 0 or 1; a is an integer ranging from 0 to 50; b is an integer which may be between 10 and 350, c is an integer ranging from 0 to 50; with the proviso that one of the parameters a and c is other than 0.

10. Composition according to Claim 9, **characterized in that** the unit of formula (I) has at least one of the following characteristics:
- the radicals G₁ denote a C₁-C₁₀ alkyl radical;
- n is non-zero and the radicals G₂ represent a divalent C₁-C₃ radical;
- G₃ represents a polymeric radical resulting from the (homo)polymerization of at least one monomer of the carboxylic acid type containing ethylenic unsaturation;
- G₄ represents a polymeric radical resulting from the (homo)polymerization of at least one monomer of the C₁-C₁₀ alkyl (meth)acrylate type.

11. Composition according to Claim 9 or 10, **characterized in that** the unit of formula (I) simultaneously has the following characteristics:
- the radicals G₁ denote a methyl radical;
- n is non-zero and the radicals G₂ represent a propylene radical;
- G₃ represents a polymeric radical resulting from the (homo)polymerization of at least acrylic acid and/or methacrylic acid;
- G₄ represents a polymeric radical resulting from the (homo)polymerization of at least methyl (meth)acrylate.

12. Composition according to any one of Claims 1 to 11, **characterized in that** the number-average molecular mass of the grafted silicone polymer ranges approximately from 10,000 to 1,000,000, and even more preferably approximately from 10,000 to 100,000.

13. Composition according to any one of the preceding claims, **characterized in that** the grafted silicone polymer(s) is (are) present in concentrations ranging from 0.01 to 20% by weight relative to the total weight of the composition, preferably from 0.1 to 15% by weight and more particularly from 0.5 to 10% by weight.

14. Composition according to any one of the preceding claims, **characterized in that** the polymer of the aqueous dispersion comprises at least one monomer chosen from styrene, butadiene, ethylene, propylene, vinyltoluene, vinyl propionate, vinyl alcohol, acrylonitrile, chloroprene, vinyl acetate, urethanes, isoprene, isobutene and esters or amides of acrylic, methacrylic, maleic, crotonic or itaconic acid, vinyl ether, vinylpyrrolidone, vinylimidazole, trimethylammonioethyl (meth)acrylate and mixtures thereof.

15. Composition according to any one of the preceding claims, **characterized in that** the nonionic polymer of the aqueous dispersion is chosen from polyesters, polyamides, polyurethanes and polyethers.

16. Composition according to any one of the preceding claims, **characterized in that** the nonionic polymer of the aqueous dispersion is chosen from:
- vinyl acetate homopolymers;
- copolymers of vinyl acetate and of acrylic ester;
- copolymers of vinyl acetate and of ethylene;
- copolymers of vinyl acetate and of maleic ester;
- vinyl chloride homopolymers;
- polyethylene waxes;
- polyethylene/polytetrafluoroethylene waxes;
- copolymers of polyethylene and of maleic anhydride;
- alkyl acrylate homopolymers and alkyl methacrylate homopolymers;
- copolymers of acrylic esters such as, for example, copolymers of alkyl acrylates and of alkyl methacrylates;
- copolymers of acrylonitrile and of a nonionic monomer chosen, for example, from butadiene and alkyl (meth)acrylates;
- styrene homopolymers;
- copolymers of styrene and of alkyl (meth)acrylate;
- copolymers of styrene, of alkyl methacrylate and of alkyl acrylate;
- copolymers of styrene and of butadiene;
- copolymers of styrene, of butadiene and of vinylpyridine;
- copolymers of styrene and of vinylpyrrolidone;
- copolymers of alkyl acrylate and of urethane.

17. Composition according to any one of Claims 1 to 14, **characterized in that** the cationic polymer of the aqueous dispersion is chosen from:
- copolymers of acrylamide and of trimethylammonioethyl (meth)acrylate;
- copolymers of alkyl methacrylate, of alkyl acrylate and of trimethylammonioethyl (meth)acrylate.

18. Composition according to any one of the preceding claims, **characterized in that** the weight concentration of the insoluble polymer particles is between 0.1 and 50% relative to the total weight of the composition.

19. Composition according to any one of the preceding claims, **characterized in that** the weight concentration of the insoluble polymer particles is between 1 and 30% relative to the total weight of the composition.

20. Composition according to any one of the preceding claims, **characterized in that** it also contains at least one additive chosen from the group consisting of thickeners, fatty acid esters, fatty acid esters of glycerol, silicones, surfactants, fragrances, preserving agents, sunscreens, proteins, vitamins, polymers, plant, animal, mineral or synthetic oils or any other additive conventionally used in the cosmetics field.

21. Composition according to any one of the preceding claims, **characterized in that** the cosmetically or dermatologically acceptable medium consists of water or a mixture of water and at least one cosmetically acceptable solvent.

22. Composition according to Claim 21, **characterized in that** the cosmetically acceptable solvents are chosen from the group consisting of monoalcohols, polyalcohols, glycol ethers, fatty acid esters and mixtures thereof.

23. Composition according to any one of the preceding claims, **characterized in that** it is in the form of a gel, a milk, a cream, a more or less thickened lotion or a mousse.

24. Composition according to any one of Claims 1 to 23, **characterized in that** it is a styling product.

25. Composition according to any one of Claims 1 to 24, **characterized in that** it is a hair product chosen from the group consisting of shampoos; rinse-out or leave-in hair products to be applied before or after shampooing, dyeing, bleaching, permanent-waving or straightening the hair.

26. Composition according to any one of Claims 1 to 25, **characterized in that** it is packaged in the form of a vaporizer, a pump-dispenser bottle or in an aerosol container in order to obtain a spray, a lacquer or a mousse.

27. Composition according to any one of the preceding claims, **characterized in that** the grafted silicone polymer is dissolved in the cosmetically or dermatologically acceptable medium or is used in the form of an aqueous dispersion of insoluble particles.

28. Non-therapeutic process for treating the hair,
**characterized in that** it consists in applying a composition as defined according to any one of Claims 1 to 27 to the said hair and then optionally in rinsing with water.
